**Europäisches Patentamt**

(19) **European Patent Office**

**Office européen des brevets**

(11) Numéro de publication: **0 084 512**
**B1**

(12) # FASCICULE DE BREVET EUROPEEN

(45) Date de publication du fascicule du brevet:
07.01.88

(51) Int. Cl.⁴: **A 61 J 1/00, A 61 M 1/00**

(21) Numéro de dépôt: **83440002.0**

(22) Date de dépôt: **07.01.83**

(54) **Poche siamoise stérile.**

(30) Priorité: **08.01.82 FR 8200758**

(43) Date de publication de la demande:
**27.07.83 Bulletin 83/30**

(45) Mention de la délivrance du brevet:
**07.01.88 Bulletin 88/1**

(84) Etats contractants désignés:
**AT BE CH DE FR GB IT LI LU NL SE**

(56) Documents cités:
**WO - A - 80/01754**
**WO - A - 83/02061**
**DE - A - 2 755 882**
**DE - B - 1 225 343**
**DE - B - 1 288 750**
**GB - A - 1 598 777**
**US - A - 3 709 222**
**US - A - 4 132 594**
**US - A - 4 259 184**
**US - A - 4 270 533**

**IBM TECHNICAL DISCLOSURE BULLETIN, vol. 18, no. 2, juillet 1975, pages 367-368, New York (USA);**

(73) Titulaire: **MACO-PHARMA, Société Anonyme, 1 rue de Metz, F-75010 Paris (FR)**

(72) Inventeur: **Goudaliez, Francis, 141 rue du Jardin des Plantes, F-59000 Lille (Nord) (FR)**

(74) Mandataire: **Lepage, Jean-Pierre, Cabinet Lepage & Aubertin Innovations et Prestations 23/25, rue Nicolas Leblanc B.P. 1069, F-59011 Lille Cédex 1 (Nord) (FR)**

## Description

La présente invention est relative à une poche siamoise stérile notamment destinée à la collecte et la conservation de fluides tels que du sang ou de ses composants. Elle trouvera tout particulièrement son application dans le domaine médical et en particulier pour les transfusions sanguines et la conservation du sang.

Actuellement, les poches sont généralement constituées par un assemblage de deux feuilles en matière plastique souple de forme identique qui sont soudées mutuellement sur la totalité de leur périmètre. Les poches sont généralement équipées d'un tube en relation avec leur volume interne qui permet l'introduction du fluide. Les poches pourront d'ailleurs être équipées de plusieurs tubes souples qui permettront d'effectuer des traitements ultérieurs.

Ces tubes souples après usage pourront être fermés hermétiquement par soudage. La récolte du fluide à l'intérieur de la poche pourra être effectuée par l'intermédiaire d'une tubulure operculée disposée sous un capuchon stérile déchirable.

Dans le cas où la poche est équipée de plusieurs tubes souples, il sera possible d'équiper certaines d'entre eux à l'aide d'un ouvre-circuit. L'ouvre-circuit est un dispositif placé à l'intérieur du tube généralement au niveau de son raccord à la poche et qui, initialement, obstrue le tube. Par conséquent, aucun écoulement ne peut être effectué par le dit tube. Les ouvre-circuits présentent en général une zone de fragilité qui permet de les briser et lorsqu'ils sont ainsi brisés, ils présentent un conduit intérieur qui autorise un écoulement du fluide et met ainsi en communication le tube avec la poche.

Le document allemand DE-B-1 225 343 montre un dispositif pour recueillir un liquide à plusieurs composants tels que le sang qui est constitué d'une poche principale reliée à une aiguille de prélèvement sanguin et d'une poche secondaire reliée à la dite poche principale par l'intermédiaire d'un capillaire. Ce dispositif permet la séparation des différents composants du sang par centrifugation.

En général, le sang prélevé aux donneurs est tout d'abord stocké dans la poche réceptrice principale. Puis par centrifugation, on sépare le sang prélevé en plasma, globules blancs et globules rouges qui se disposent en couches dans la poche principale.

La poche principale est ensuite mise en communication avec une première poche auxiliaire dans laquelle on expulse le plasma qui surnageait dans la poche principale. La poche, contenant le plasma peut être fermé hermétiquement par soudage du tube qui sera également sectionnée afin de séparer cette première poche auxiliaire qui sera acheminée vers un lieu de stockage approprié.

Par ailleurs, la poche principale peut être reliée à une seconde poche auxiliaire notamment par l'intermédiaire de tubes en Y. En outre, la seconde poche auxiliaire peut présenter deux compartiments.

Actuellement, cette poche auxiliaire à deux compartiments est constituée par l'assemblage de deux feuilles de forme identique, soudées d'une part sur la totalité de leur périphérie, et d'autre part dans leur partie centrale suivant une zone localisée d'étendant entre les bords des feuilles. La poche auxiliaire présente ainsi l'aspect de deux poches indépendantes ayant un bord commun constitué par la zone localisée de soudure.

Les globules blancs sont introduits dans l'un des compartiments de la seconde poche secondaire afin d'y être stockés. Comme pour la première poche auxiliaire, le tube de liaison sera soudé hermétiquement et coupé. Le deuxième compartiment de la seconde poche auxiliaire contient une solution de SAG qui est un composé de chlorure de sodium, adénine et glucose, ayant la propriété de permettre la conservation des globules rouges. En effet, les globules rouges ont habituellement une durée de vie de 21 jours qui pourrait être prolongée jusqu'à 35 jours par addition de SAG.

La solution de SAG est donc retransférée dans la poche principale pour y être mélangée avec les globules rouges afin que ceux-ci conservent. La poche principale peut être fermée hermétiquement par soudage de son tube de liaison et en sectionnant le tube, il est ainsi possible de disposer de trois poches, la poche principale contenant des globules rouges additionnés de SAG, une première poche auxiliaire contenant du plasma et une seconde poche auxiliaire dont un des compartiments contient des globules blancs, l'autre compartiment étant vide.

Ainsi, à l'issue des opérations de séparation des composants du sang, il subsiste une seconde poche auxiliaire dont l'un des compartiments est vide et qui devient donc inutile.

La poche à deux compartiments précédemment décrite présente un inconvénient majeur qui provient du fait que la séparation entre les deux compartiments est fixe. Aussi, chacun des compartiments ne peut contenir qu'une quantité déterminée de sang ou de ses composants, imposée par la position de la soudure centrale. Cela est gênant dans certaines utilisations lorsque les deux compartiments doivent contenir des volumes très inégaux.

Par ailleurs, il est connu deux brevets, à savoir le brevet DE-B-1 288 750 et le brevet US-A-4 270 533 qui montrent des systèmes, voisins l'un de l'autre, destinés au stockage et à la perfusion de liquides tels que notamment le sang.

Ces systèmes présentent une poche souple comprenant deux compartiments séparés par une cloison centrale qui s'étend contre les parois extérieures. L'un des compartiments est rempli préalablement du liquide à perfuser et l'autre compartiment est relié à une source d'air comprimé afin d'exercer une compression sur le compartiment qui contient le liquide en vue de contrôler le débit du liquide lors de la perfusion.

Toutefois, cette poche double ne peut pas être utilisée comme moyen de collecte et de stockage de fluide tel que le sang ou ses composants étant donné notamment que l'un de ses compartiments est conçu spécialement pour contenir de l'air comprimé.

Le but principal de la présente invention est de proposer une poche siamoise stérile, destinée à la collecte et à la conservation de fluide tel que le sang ou ses composants, dont on peut modifier le volume de stockage de chacun des compartiments. A cet effet, dans la poche selon l'invention, les compartiments seront séparés par une cloison mobile qui permettra d'accroître les possibilités de stockage de l'un ou de l'autre des compartiments.

La poche siamoise stérile selon l'invention permet de standardiser la fabrication des poches à deux compartiments, car elle peut être utilisée pour différents volumes de stockage sans qu'il soit nécessaire de modifier la fixation de la cloison mobile.

Un autre but de la présente invention est de proposer une poche siamoise présentant plusieurs compartiments qui sont séparés par des parois intérieures présentant des propriétés particulières autorisant, par exemple, les échanges gazeux ou la dialyse.

D'autres buts et avantages de la présente invention apparaîtront au cours de la description qui va suivre, qui n'est cependant donnée qu'à titre indicatif et qui n'a pas pour but de la limiter.

Selon l'invention, la poche siamoise stérile, destinée à la collecte et à la conservation de fluide tel que le sang ou ses composants, formant un élément distinct indépendant, comprenant extérieurement deux feuilles souples jointes sur leur périmètre, présentant intérieurement au moins deux compartiments, les compartiments étant séparés par une feuille intermédiaire formant une paroi qui peut se déplacer et s'étendre à plat contre au moins une des feuilles extérieures, la souplesse des feuilles permettant de modifier le volume de stockage de chacun des compartiments indépendamment l'un de l'autre, l'un des compartiments destiné à recevoir les fluides à conserver étant pourvu d'une tubulure operculée et d'une autre tubulure ouverte, et au moins un autre deuxième compartiment présentant une tubulure, est caractérisée en ce que la tubulure de ce deuxième compartiment, qui est destiné à recevoir un liquide de conservation, est munie d'un ouvre-circuit présentant une zone de fragilité qui permet de le briser de manière à mettre en communication la tubulure avec le dit deuxième compartiment.

Par ailleurs, l'invention propose un dispositif, destiné d'une part à la séparation stérile des composants du sang par centrifugation, tels que le plasma, les globules blancs et les globules rouges, et d'autre part à l'utilisation séparée de chacun des composants, le dit dispositif étant constitué par:

– une première poche présentant au moins un compartiment destiné à recueillir le sang avec ses différents composants par ponction veineuse,

– une poche auxiliaire présentant au moins un compartiment destiné à recueillir le plasma avec centrifugation de la dite première poche,

– une deuxième poche, présentant au moins un compartiment destiné à recevoir les globules blancs,

– une liaison stérile entre la première poche, la poche auxiliaire et la deuxième poche,

est caractérisé par le fait que la dite deuxième poche est constituée par une poche siamoise selon l'invention, telle que définie ci-dessus, le deuxième compartiment de cette poche siamoise étant destiné à contenir une solution de conservation.

L'invention sera mieux comprise si l'on se réfère à la description ci-dessous, ainsi qu'au dessin en annexe qui en fait partie intégrante.

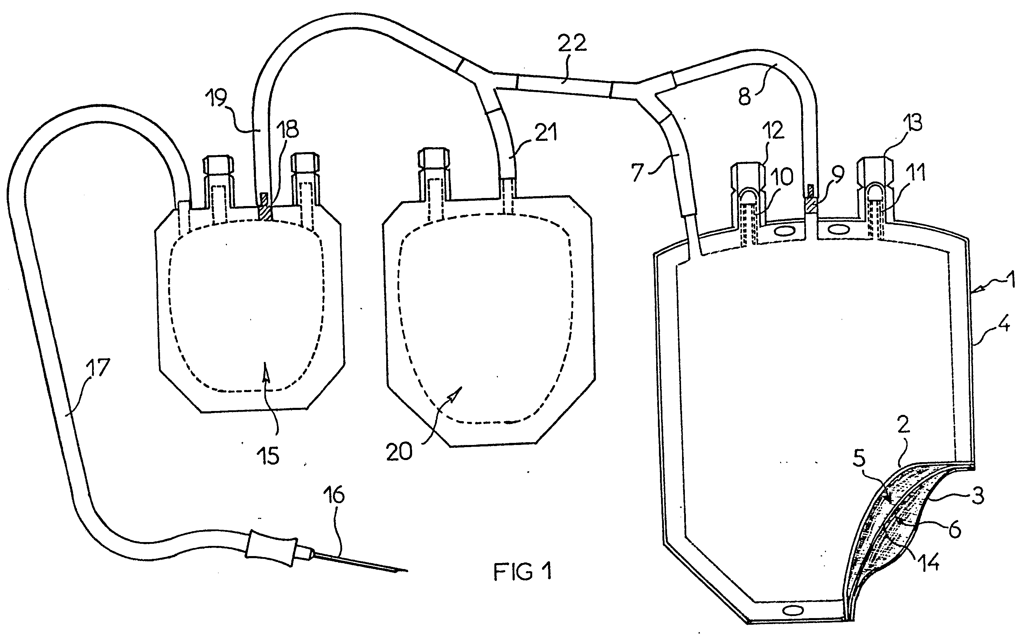

La figure 1 schématise une poche siamoise dans un mode préférentiel de réalisation de l'invention, installée dans un ensemble destiné à recueillir les composants du sang obtenus par centrifugation.

A la figure 1 est représentée sous le repère 1 une poche siamoise selon l'invention. Elle servira à la collecte et la conservation de fluides tels que le sang ou ses composants.

Généralement, les poches sont réalisées en un matériau souple étanche. La poche est constituée extérieurement de deux feuilles souples 2 et 3, généralement de mêmes dimensions et jointes sur leur périmètre 4. La jointure est en général réalisée de manière étanche par soudage.

La poche siamoise comprend au moins deux compartiments 5 et 6, visibles sur la coupe partielle de la figure 1. Chaque compartiment 5 et 6 sera équipé d'au moins un tube, respectivement 7 et 8, en relation avec les compartiments qui permettra l'introduction du fluide dans les compartiments.

Ces tubes seront de préférence réalisés dans un tuyau souple qui pourra être obturé hermétiquement par soudage. Ces tubes pourront être équipés d'ouvre-circuits qui, à l'origine, obstruent hermétiquement le conduit des tubes. Ces ouvre-circuits présentent une zone de fragilité qui permet de les briser, généralement par pliage et lorsqu'ils sont brisés, ils présentent un conduit intérieur qui met en communication le tube avec la poche.

Dans le mode de réalisation de la figure 1, le tube 8 est équipé d'un tel ouvre-circuit 9 qui présente une zone de fragilité, permettant de le briser, et qui, lorsqu'il est brisé, met en communication le tube 8 avec le compartiment 6 de la poche siamoise 1.

Les compartiments 5 et 6 présentent également au moins une tubulure operculée, respectivement 10 et 11, stérile et protégée par un capuchon déchirable 12 et 13. Ces tubulures operculées permettront d'extraire ultérieurement, le contenu des compartiments de la poche siamoise.

Selon l'invention, les compartiments 5 et 6 seront séparés par une feuille intermédiaire 14, visible dans la coupe partielle de la figure 1, formant une paroi qui s'étend à plat contre au moins une feuille extérieure 2 ou 3.

Dans le cas de la figure 1, la feuille intermédiaire 14 s'étend sur toute la surface des feuilles extérieures 2 ou 3, mais il aurait pu également être envisagé que cette feuille 14 présente une surface inférieure à celle des feuilles 2 et 3 et qu'elle soit assemblée à plat contre l'une des feuilles par fixation de sa périphérie sur la surface de l'une des feuilles.

Il pourra être également envisagé une pluralité de compartiments qui seraient chacun séparé par une feuille disposée à plat entre les deux feuilles extérieures.

Généralement, une certaine étanchéité est recherchée entre les différents compartiments et par conséquent il sera préférable de fixer la feuille intermédiaire de façon étanche au niveau de son périmètre.

Si l'on désire obtenir deux compartiments de volumes sensiblement égaux, ce qui est généralement le cas, la feuille intermédiaire 14 présentera sensiblement les mêmes dimensions que les feuilles extérieures 2 et 3 et sera fixée par son périmètre au niveau de la jointure 4 des deux feuilles extérieures.

La feuille intermédiaire 14 pourra également présenter une zone de fragilité, que l'on pourra rompre facilement et qui, après rupture, mettra en communication les compartiments contigus.

De plus, il pourra également être utilisé une feuille intermédiaire 14 qui soit semi-perméable autorisant ainsi par exemple les échanges gazeux ou la dialyse.

De même, il pourra être utilisé une feuille intermédiaire 14 qui ne soit pas réalisée dans le même matériau que les feuilles extérieures ou qui présente des propriétés particulières.

Le mode de fixation préférentiel de la feuille 14 sur la feuille extérieure 2 ou 3 sera le soudage ou le collage.

La figure 1 représente un ensemble de poches destinées à la collecte et la séparation des composants du sang obtenus par centrifugation.

Le sang est tout d'abord recueilli dans la poche principale 15 par l'intermédiaire d'une aiguille creuse 16 et d'un tube souple 17 selon les procédés de prélèvement connus. Ensuite, le tube 17 est fermé hermétiquement par soudage, puis sectionné afin de retirer la partie comportant l'aiguille devenue inutile.

La poche 15 subit ensuite une centrifugation afin de séparer les composants du sang, à savoir le plasma, les globules blancs et les globules rouges. L'ouvre-circuit 18 du tube 19 de la poche 15 est ensuite brisé afin de diriger le plasma dans la première poche auxiliaire 20 par l'intermédiaire du tube 19 raccordé au tube 21, en communication avec la poche 20. Puis, le tube 21 peut être fermé hermétiquement par soudage et sectionné libérant ainsi la poche 20 de l'ensemble.

Les globules blancs peuvent ensuite être dirigés dans le premier compartiment par exemple de la poche siamoise 1 par l'intermédiaire du tube 19, de la section 22 et du tube 7. Puis, le tube 7 peut être obturé hermétiquement par soudage et sectionné.

Ensuite, l'ouvre-circuit 9 pourra être brisé afin d'injecter par l'intermédiaire du tube 8, de la section 22 et du tube 19, la solution de SAG contenue initialement dans le second compartiment de la poche siamoise 1, dans la poche 15 où il ne reste que les globules rouges. Le tube 8 peut ensuite être fermé hermétiquement et sectionné ainsi que le tube 19. A l'issue de l'opération, il sera obtenu trois poches contenant respectivement les globules rouges additonnés de SAG, le plasma et les globules blancs.

La surface extérieure de la poche siamoise 1 correspondra exactement avec le volume de son contenu, la surface inutilisée des poches à plusieurs compartiments antérieurs est ainsi supprimée.

**Revendications**

1. Poche siamoise stérile (1) destinée à la collecte et à la conservation de fluides tels que le sang ou ses composants, formant un élément distinct indépendant, comprenant extérieurement deux feuilles souples (2, 3) jointes sur leur périmètre (4), présentant intérieurement au moins deux compartiments (5, 6), les compartiments étant séparés par une feuille intermédiaire (14) formant une paroi qui peut se déplacer et s'étendre à plat contre au moins une des feuilles extérieures (2, 3), la souplesse des feuilles (2, 3, 14) permettant de modifier le volume de stockage de chacun des compartiments indépendamment l'un de l'autre, l'un (5) des compartiments (5, 6) destiné à recevoir les fluides à conserver étant pourvu d'une tubulure operculée (10) et d'une autre tubulure ouverte (7), et au moins un autre deuxième compartiment (6) présentant une tubulure (8), caractérisée en ce que la tubulure (8) de ce deuxième compartiment (6), qui est destiné à recevoir un liquide de conservation, est munie d'un ouvre-circuit (9) présentant une zone de fragilité qui permet de le briser de manière à mettre en communication la tubulure (8) avec le dit deuxième compartiment (6).

2. Poche siamoise stérile selon la revendication 1, caractérisée par le fait que la feuille intermédiaire (14) présente une zone de fragilité qui, après rupture, autorise des échanges entre les compartiments (5, 6) contigus.

3. Dispositif, destiné d'une part à la séparation stérile des composants du sang par centrifugation, tels que le plasma, les globules blancs et les globules rouges, et d'autre part à l'utilisation séparée de chacun des composants, le dit dispositif étant constitué par:

– une première poche (15) présentant au moins un compartiment destiné à recueillir le sang avec ses différents composants par ponction veineuse,

– une poche auxiliaire (20) présentant au moins un compartiment destiné à recueillir le plasma après centrifugation de la première poche (15),

– une deuxième poche, présentant au moins un compartiment (5) destiné à recevoir les globules blancs,

– une liaison stérile entre la première poche (15), la poche auxiliaire (20) et la deuxième poche, caractérisé par le fait que la dite deuxième poche est constituée par une poche siamoise (1) selon la revendication 1, le deuxième compartiment (6) de cette poche siamoise étant destiné à contenir une solution de conservation.

## Claims

1. A sterile siamesed pocket (1) adapted to the collecting and the conservation of fluids such as the blood or its components, forming an independent distinct element, comprising externally two flexible sheets (2, 3) joined to their perimeter (4), having internally at least two compartments (5, 6), the compartments being separated by an intermediate sheet (14) forming a wall which can shift and extend flat against at least one of the outer sheets (2, 3), the flexibility of the sheets (2, 3, 14) permitting to modify the storage volume of each of the compartments independently of each other, one of the compartments (5, 6) adapted to receive the fluids to be conserved being provided with an operculated tubulure (10) and an other open tubulure (7), and at least an other second compartment (6) having a tubulure (8), characterized in that the tubulure (8) of this second compartment (6), which is adapted to receive a liquid of conservation, is provided with a circuit-opener (9) having a fragility zone which allows to break it in order to put into communication the tubulure (8) with said second compartment (6).

2. A sterile siamesed pocket according to claim 1, characterized by the fact that the intermediate sheet (14) comprises a fragility zone which, after the rupture, authorizes exchanges between the contiguous compartments (5, 6).

3. A device adapted to, on the one hand, the sterile separation of the blood components by centrifugation, such as the plasma, the white globules and the red globules and, on the other hand, the separated utilisation of each of the components, said device being composed of:

– a first pocket (15) comprising at least a compartment adapted for collecting the blood with its different components by veinous puncture,

– an auxiliary pocket (20) presenting at least a compartment adapted for collecting the plasma after centrifugation of the first pocket (15),

– a second pocket comprising at least a compartment (5) adapted for collecting the white globules,

– a sterile linkage between the first pocket (15), the auxiliary pocket (20) and the second pocket, characterized by the fact that said second pocket is composed of a siamesed pocket (1) according to claim 1, the second compartment (6) of this siamesed pocket being adapted to contain a solution of conservation.

## Patentansprüche

1. Steriler siamesischer Beutel (1) zur Aufnahme und Aufbewahrung von Flüssigkeiten, wie beispielsweise Blut oder seine Bestandteile, der eine getrennte, unabhängige Einheit bildet, und der aussen zwei flexible Folien (2, 3) aufweist, die auf ihrem Umfang (4) miteinander verbunden sind, und innen mindestens zwei Kammern (5, 6) aufweist, die durch eine Zwischenfolie (14) getrennt sind, die eine Wand bildet, die beweglich ist und an mindestens einer der äusseren Folien (2, 3) flach anliegen kann, wobei die Flexibilität der Folien (2, 3, 14) gestattet, das Aufnahmevolumen jeder Kammer unabhängig voneinander zu verändern, und die eine Kammer (5) der Kammern (5, 6), die für die Aufnahme der aufzubewahrenden Flüssigkeiten bestimmt ist, mit einem durch einen Deckel verschliessbaren Rohrstutzen (10) und einem weiteren, offenen Rohrstutzen (7) versehen ist, und mindestens eine weitere, zweite Kammer (6) einen Rohrstutzen (8) aufweist, dadurch gekennzeichnet, dass der Rohrstutzen (8) dieser zweiten Kammer (6), die dazu bestimmt ist, eine Konservierungsflüssigkeit aufzunehmen, mit einer Sperrwand (9) versehen ist, die eine zerbrechliche Zone aufweist, um diese Sperrwand (9) durchbrechen zu können, so dass der Rohrstutzen (8) mit der besagten zweiten Kammer (6) verbunden wird.

2. Steriler siamesischer Beutel gemäss Anspruch 1, dadurch gekennzeichnet, dass die Zwischenfolie (14) eine zerbrechliche Zone aufweist, um diese Zwischenfolie (14) durchbrechen zu können, so dass ein Flüssigkeitsaustausch zwischen den aneinander angrenzenden Kammern (5, 6) ermöglicht wird.

3. Vorrichtung, einerseits zur sterilen Trennung, durch Zentrifugieren, der Bestandteile des Bluts, wie beispielsweise Plasma, weisse Blutkörperchen und rote Blutkörperchen, und andererseits zur getrennten Verwendung von jedem der Bestandteile, wobei die besagte Vorrichtung gebildet wird von:

– einem ersten Beutel (15) mit mindestens einer Kammer, die dazu bestimmt ist, das durch Punktion aus einer Vene entnommene Blut aufzunehmen;

– einem Hilfsbeutel (20) mit mindestens einer Kammer, die dazu bestimmt ist, das Plasma aufzunehmen, das beim Zentrifugieren des Blutes aus dem ersten Beutel erhalten wird;

– einem zweiten Beutel mit mindestens einer Kammer (5), die dazu bestimmt ist, die weissen Blutkörperchen aufzunehmen;

– einer sterilen Verbindung zwischen dem ersten Beutel (15), dem Hilfsbeutel (20) und dem zweiten Beutel, dadurch gekennzeichnet, dass der besagte zweite Beutel ein siamesischer Beutel (1) gemäss dem Anspruch 1 ist, wobei die zweite Kammer (6) dieses siamesischen Beutels dazu bestimmt ist, eine Konservierungslösung aufzunehmen.

FIG 1

0 084 512